# EUROPEAN PATENT SPECIFICATION

(11) **EP 1 315 423 B1**
(45) Date of publication and mention of the grant of the patent: **31.03.2010**
(21) Application number: 01963628.1
(22) Date of filing: 22.08.2001
(51) Int. Cl.: A01N 63/00, A01N 25/22, A61K 47/36, C12N 1/00

(54) **A THERMO-STABLE BIO-MATRIX**
THERMOSTABILE BIOMATRIX
MATRICE BIOLOGIQUE THERMOSTABLE

(30) Priority: 22.08.2000 NZ 50648400; 22.08.2000 NZ 50648500
(43) Date of publication of application: 04.06.2003
(73) Proprietor: Encoate Holdings Limited, Hamilton (NZ)
(72) Inventor: JOHNSON, Von, Walter, Christchurch (NZ); PEARSON, Joan, Frances, Christchurch (NZ)
(74) Representative: Wilson Gunn
(86) International application number: PCT/NZ2001/000167
(87) International publication number: WO 2002/015702

(56) References cited:
- EP-B- 0 234 670
- WO-A-92/20229
- US-A- 5 021 350
- US-A- 5 292 507
- US-A- 5 401 506
- US-A- 5 441 735
- US-A- 5 670 368
- US-A- 5 686 385
- US-A- 5 916 029
- PATENT ABSTRACTS OF JAPAN & JP 62 234 005 A (SEIKAKEN KK) 14 October 1987

## Description

### TECHNICAL FIELD

The present invention relates to a process and product for the stabilisation and storage of biological materials, wherein these biological materials are micro-organisms. More particularly the present invention reflates to a process for producing a bio-polymer matrix for the stabilisation and storage of such materials.

### BACKGROUND ART

A know problem associated with the industrial or agricultural application of biological materials is the maintenance of the materials in a viable state or a stable state until they are used, or during the period of time they may be incorporated in a slow release delivery mechanism. Many biological materials cannot be maintained in a viable condition during storage, particularly where they are not kept or can not be kept under refrigeration. This is a particular problem with non spore forming bacteria.

At present, use of bacterial products as the biological material requires production of high concentrations of bacteria to ensure survival of commercially useful numbers for extended periods. This has been achieved to a limited degree using refrigeration and/or freeze drying to preserve viability. Additionally, while some microbial products require only the delivery of an inoculative dose, for others (such as bio-pesticides), delivery of a higher minimum dosage concentration is essential to the success of the product.

A number of different formulations and media have been proposed, used and disclosed in order to overcome this "shelf-life" problem. Some formulations emphasise the selection of the basic active ingredient for the storage matrix "the bio-polymer", whilst others disclose methods for preparation of this matrix, or the method of introduction of the biological agent into the matrix and the conditions under which any of these steps occur.

### CONDITIONS

WO98/13471 discloses a formulation formed from polyvinylpyrrolidone (PVP). With the use of this active ingredient as the matrix, some biological material is found to survive for at least 8 ½ months when stored in vacuum packaging in a temperature range of 5-25°C.

US Patent No. 4434231 discloses a polymer matrix which is partially cross-linked and comprised of a gel of one or more polymers. The gel is dried and it was found that the biological agent was not converted to a dormant or latent state. The partially cross-linked polymer is effected by one of the following: heat treatment, metallic salt action, introduction of a further polymer or another polysaccharide. Additionally, it discloses the gel as being prepared at elevated temperatures, prior to the introduction of the liquid culture of the biological agent. Further complexity is added in the examples disclosed to show the viability of the one selected organism, *Rhizobium japonicum*.

WO98/13471 also discloses that vacuum packaging significantly decreases the practicality of the commercial production of the product.

### FORMULATIONS

US Patent No. 4155737 discloses the use of the polymer, polyacrylamide. Use of xanthan, carob, carrageenan, and sodium alginate is disclosed in US Patent Nos. 4434231, 5021350, 5292507. WO98/13471 discloses PVP as enhancing survival of sensitive micro-organisms.

US Patent 5292507 discloses and addresses the necessity for additional steps and preparation of gels to avoid the handling that is attendant on use of such gums as xanthan gum. However, this patent discloses only the use of a liquid system which specifically avoids semi-solids, viscous gels or have gum-like properties. It discloses the use of polysaccharide and polymers that are not cross-linked or are not substantially cross-linked where the degree of cross-linking is less than 10%.

US Patent 5292507 discloses a method for suspending bacterial cells in a non cross-linked polysaccharide solution, and incorporation into an oil emulsion. This solution is then diluted with water and used in a liquid spray either for direct application or for coating of seeds. The solution may also be reduced to a powder. Finally, the liquid solution prepared has by weight 0.05% to 10% non cross-linked polysaccharide.

US Patent 5113619 discloses a composition which includes bacteria and an adherent which is a bio-polymer. The bio-polymer acts as a matrix for protecting the bacteria, which is applied to a seed.

As can be seen from the preceding patents, many disclose the advantageous use of two bio-polymers. However such use adds to the handling costs, leading to a more expensive production technique than the use of a single bio-polymer.

The above discussed matrices formed in pourable liquids also require that transportation costs are higher than they might otherwise be. Further, processing treatments to the liquid are also higher than are needed.

### OTHER PREPARATION METHODS

Other two-stage processes for deriving a matrix for stable storage of biological agents can be found in US Patent No. 4954443. This discloses the use of a first and a second aqueous solution for the immobilisation of enzymes and micro-organisms. The first solution contains at least one immobilising agent, which can be xanthan gum or its derivatives. The second aqueous solution includes metal ions having a valence of three or more. After the two solutions are combined the immobilising agent is thereby hardened into a state in which it encloses the biological agents.

However, as with previous methods of producing biological storage medium, non-biodegradable or toxic elements are introduced into the process to form the storage medium. Further, this invention does not disclose any survival rates of micro-organisms and thus may not be useful for agricultural or environmental applications, especially with respect to bio-inoculants.

It is an object of the present invention to provide a process for producing a storage medium for biological materials, wherein these biological materials are micro-organisms, which is simple, easy to effect, and produces a non-toxic bio-degradable matrix, without reducing the efficiency of the storage, stabilisation or preservative characteristics of the bio-matrix at room temperature and pressure. ,

It is a further object of the present invention to address the foregoing problems or at least to provide the public with a useful choice.

Further aspects and advantages of the present invention will become apparent from the ensuing description which is given by way of example only.

### DISCLOSURE OF INVENTION

According to one aspect of the present invention there is provided a method for producing a thermo-stable bio-degradable medium for storage of biological materials, said method including the steps of:
(a) preparing at least one bio-polymer at a concentration of 100-10% by weight of a mixture at room temperature, said mixture being in a state selected from a solid and a suspension;
(b) preparing a concentrate of the biological materials of between 10% and 100% (by weight), said concentrate being suspension;
(c) combining the mixture of step (a) and the preparation (b), to form a second mix; and
(d) agitating the second mix at room temperature to form a homogeneous suspension; wherein a gel is formed;
and wherein the bio-polymer is selected from the group: xanthan gum; acacia gum; guar gum; gellan; starch; and a combination thereof;
and wherein the biological material is a micro-organism.

According to a further aspect of the present invention there is provided a method of producing a thermo-stable biodegradable medium as described above wherein within step (c) the ratio of the mixtures of steps (a) and (b), which are combined in step (c), is in the range 1:10 to 10:1 by weight. The range is optimally 1:1.

According to a further aspect of the present invention there is provided a method of producing a thermo-stable biodegradable medium as described above wherein said biological material is between 10 to 20% by weight in the concentrate of step (b).

Optimally, the second mix should be allowed to stand at room temperature after step (d) and the time should be approximately 60 minutes since being made.

According to a further aspect of the present invention there is provided a method for producing a thermo-stable bio-degradable medium for storage of biological materials wherein said method includes, a further step, before step (d):
(ci) adding a bio-degradable non-toxic oil to the mix, the concentration of oil being in the range 0.1 to 10% by weight of the mix.

Optimally also, the oil is in the range of 1% to 10% by weight of the mix.

Optionally, the oil used in step (d) may be any biodegradable, monounsaturated oil which can be used in a refined or non-refined state. The oil may include a combination of oils, which may or may not be edible, as is desired For example, olive oil, canola oil, sunflower seed oil, and hydrolysed oils may be used as is desired.

In the method of producing a thermo-stable biodegradable medium as described above, the concentration of biological material, at the end of step (d) is hereafter referred to as the "cell concentration". Advantageously, the cell concentration is in the range 10⁵ cells to 10¹² cells g⁻¹, more preferably in the range 10⁸ to 10¹² cells g⁻¹, more preferably in the range of 10⁹ to 10¹⁰ cell g⁻¹. Advantageously, the biological material may be present in the concentrate of step (b) in a broth, or on a growing medium.

According to another aspect of the present invention the micro-organism is selected from the group: *Serratia, Pseudomonas, Xanthomonas* and *Rhizobium*, and a combination thereof.

Optionally, the bio-polymer is xanthan gum, or a mixture of xanthan and acacia gums, which is added as a dry solid in a ratio in the range of 1:2 to 1:6 by weight

It will be appreciated that more than one bio-polymer and/or more than one biological agent may be present in the steps (a) to (c) as described above.

According to a further object of the present invention there is provided a method of producing a thermo-stable biodegradable medium as described above wherein said method includes: the steps of (a) to (c) with at least one first bio-polymer; the steps of (a) to (c) with at least one second bio-polymer; and a mixing of these two mixtures by steps (c) and (d) as described above.

For the purposes of the specification, the term "storage" means a stability of better than LT₅₀ with respect to the cell concentration of the biological material. That is, more than 50% of the cells (if cells are the biological material) are viable at the end of the storage period; or more than 50% of the non-living material is viable at the end of the storage period. Advantageously, LTso may be achievable after 2 months, 4 to 6 months, or 12 to 18 months.

For the purposes of the specification the term "thermo-stable" means a range of temperatures in which the combination of bio-polymer and biological material is stable. This temperature range is 4°C to 40°C preferably between 5°C to 30°C.

According to a further aspect of the present invention there is provided the method as described above wherein said method includes a further step (e):
(e) spreading the gel to 5-10 mm in thickness and air-drying it to a moisture content in the range 0.05% to 20% by weight.

Optionally, step (e) takes from 12 to 17 hours at ambient or room temperature. Optionally also, the gel is at a thickness of 5 mm, before drying. Optionally the moisture content is approximately 20% by weight at the end of the drying.

For the purposes of this specification the term "substrate" is used to encompass, but is not limited to, agricultural, horticultural, forestry or other commercial substrates, such as grasses and crops, soils (etc); water, waste water, skins of animals and tissues of animals; and solids such as sands and gravels and other uncultivated and friable materials.

It can be seen from the above described invention that storage of a biological material can be effected without the need for special conditions, and after a sample preparation process for the bio-matrix used as the storage medium.

### BEST MODES FOR CARRYING OUT THE INVENTION

### Example 1

For each micro-organism test below, 7.5 grams of dried xanthan gum is added to 135 grams of concentrated biological material by agitation at room temperature to form a homogenous mix.

This mix is left for 1 hour at room temperature. 7.5 grams of pure canola oil is added and the suspension is agitated for 10-15 minutes at room temperature.

A gel is made with the bio-polymer xanthan and one of each of a range of micro-organisms: *Serratia entomophila*, *Serratia marcescens*, *Pseudomonas*. *aeruginosa*, *Rhizabium leguminosarum* (biological materials). Each of these micro-organisms is at a call concentration of approximately 10⁹ to10¹⁰ cells g⁻¹. The cell concentrations are set out in Table 1.

The survival rate of the micro-organisms is tested and the results are set out in Table 1.

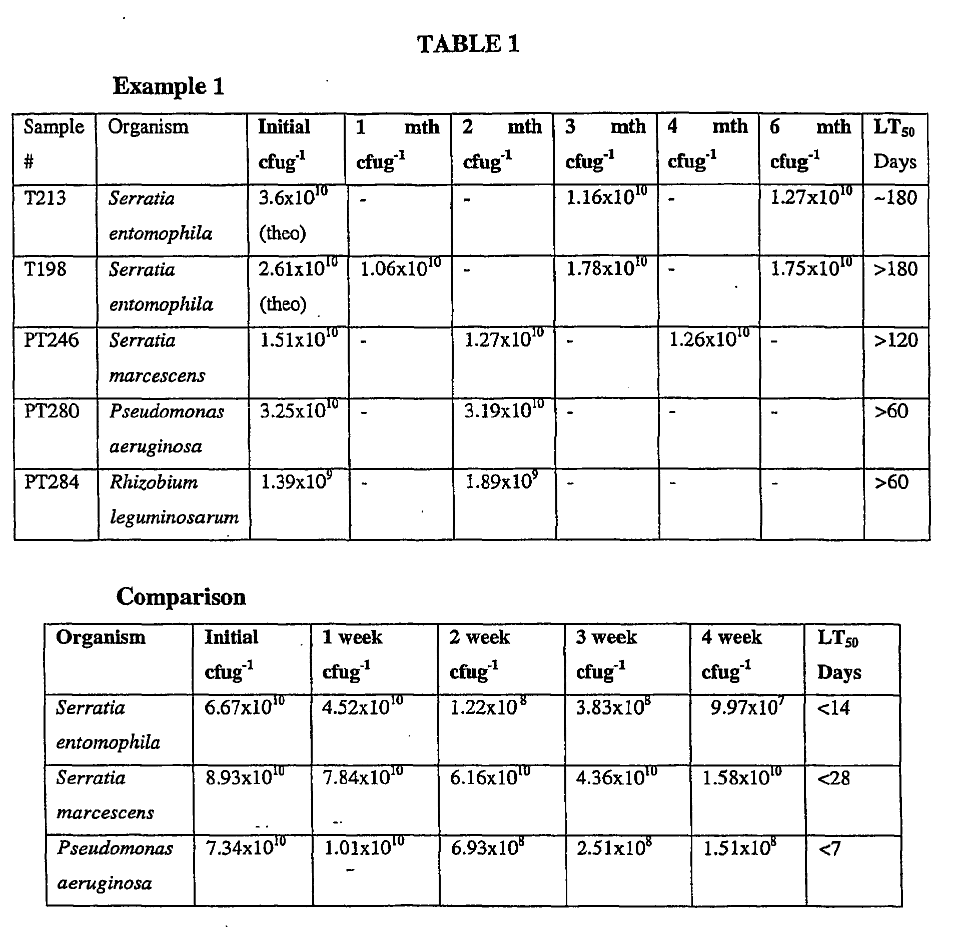

A further example is also included, T213, using the above method wherein 5 grams of xanthan gum, 5 grams of oil and 90 grams of concentrated biological material containing *Serratia entomophila* are used.

By comparison, tests were done showing the rate of microbe survival for the microbe in a broth at 20° C. The results are shown above in Table 1 under the heading comparison.

### Example 2

Separate gels are made with the bio-polymer starch and one each of a range of micro-organisms: *Serratia marcescens, Pseudomonas aeruginosa, Rhizobium leguminosarum* (biological materials). Each of these micro-organisms is concentrated at approximately 10¹⁰ cells g⁻¹. The cell concentrations are set out in Table 2.

For each composition 15 grams of starch is added to 100 grams of microbial concentrate. The mix is agitated for 10 minutes at room temperature. The resultant gel matrix is stored in a plastic bag at a shelf temperature of approximately 20°C for up to 2 months.

The survival rate of the micro-organisms were tested and the results are shown in the attached Table 2.

**TABLE 2**

| **Example 2** | | | | |
|---|---|---|---|---|
| **Sample** | **Organism** | **Initial cfu g⁻¹** | **2 month cfu g⁻¹** | **LT₅₀** Days |
| PT248 | *Serratia marcescens* | 1.89x10¹⁰ | 3.45x10¹⁰ | >60 |
| PT282 | *Pseudomonas aeruginosa* | 2.89x10¹⁰ | 1.27x10¹⁰ | -60 |
| PT286 | *Rhizobium leguminosarum* | 6.26x10⁸ | 1.28x10⁹ | >60 |

### Example 3

Separate gels are made with the bio-polymer xanthan and one of each of a range of micro-organisms: *Serratia entomophila, Serratia marcescens, Pseudomonas aeruginosa* (biological materials). Each of these micro-organisms is concentrated at approximately 10¹⁰ cells g⁻¹. The cell concentrations are set out in Table 3.

For each sample, to 7.5 grams dry xanthan gum is added 42.5 grams distilled water. The mixture is agitated at room temperature for between 5-10 minutes to form a suspension. Alternatively a 50% solution of xanthan gum medium may be used. 50 grams of each micro-organism concentrate is added to the respective suspension. The mix is agitated for a further 10 minutes at room temperature. The resultant gel matrix is stored in a plastic bag at a shelf temperature of approximately 20°C for up to 2 months.

The survival rate of the micro-organisms are tested and the results are set out in the attached Table 3.

**TABLE 3**

| **Example 3** | | |
|---|---|---|
| **Organism** | **Initial Concentration cfu g⁻¹** | **Survival - 2 months cfu g⁻¹** |
| *Serratia entomophila* | 3.32x10¹⁰ | 1.51x10¹⁰ |
| *Serratia marcescens* | 4.47x10¹⁰ | 1.73x10¹⁰ |
| *Pseudomonas aeruginosa* | 1.05x10¹⁰ | 7.63x10¹⁰ |

### Example 4

The gels from Example 1 are each spread out to a 5 mm thickness and air dried at room temperature for 15-20 hours. The dry gels are each stored in a plastic container at room temperature for up to 6 months.

The survival rate of the micro-organisms are tested and the results are set out in the attached Table 4.

**TABLE 4**

| **Example 4** | | | |
|---|---|---|---|
| **Organism** | **Initial Concentration cfu g⁻¹** | **Survival 1 month cfu g⁻¹** | **Survival 2 months cfu g⁻¹** |
| *Serratia entomophila* | 7.03x10¹⁰ | 7.99x10⁸ | - |
| *Serratia marcescens* | 1.27x10¹⁰ | 8.36x10⁸ | 3.62x10⁷ |
| *Pseudomonas aeruginosa* | 8.30x10¹⁰ | 3.26x10¹⁰ | 4.95x10⁹ |
| *Xanthomonas campestri* | 5.69x10⁹ | 7.68x10¹⁰ | 4.91x10¹⁰ |

### Example 5

For each micro-organism a suspension of bio-polymer was prepared as follows: 4 grams of dried xanthan gum was added to 21 grams of water. (Alternatively a 50% suspension of xanthan gum was used.) 25 grams of concentrated biological material (as described for each micro-organism from example 1) was added to this suspension and agitated at room temperature. This was left at room temperature for between 1/2 an hour to an hour.

At the same time 11 grams of acacia gum were added to 14 grams of water and a suspension formed after agitation at room temperature. 25 grams of concentrated biological material was added at room temperature and agitated. This was also kept for 30 to 60 minutes at room temperature after agitation.

The two separate mixtures were added together and kept at room temperature to form a homogenous mix. This solution was kept at room temperature for 1.5 to 2.5 hours, after which a gel was formed. Each gel matrix was stored in plastic bottles at room temperature.

The survival rate of the micro-organisms were tested and the results are set out in the attached Table 5.

**TABLE 5**

| **Example 5** | | | |
|---|---|---|---|
| **Organism** | **Initial Concentration cfu g⁻¹** | **Survival 1 month cfu g⁻¹** | **Survival 2 months cfu g⁻¹** |
| *Serratia entomophila* | 5.10x10¹⁰ | 2.22x10⁷ | 1.17x10⁵ |
| *Serratia marcescens* | 2.38x10¹⁰ | 2.76x10⁷ | 5.81x10⁶ |
| *Pseudomonas aeruginosa* | 1.01x10¹⁰ | 3.71x10⁸ | 3.51x10⁹ |
| *Xanthomonas campestri* | 2.83x10¹⁰ * | 6.86x10¹⁰ | 1.51x10⁸ |

| | | | |
|---|---|---|---|
| * = theoretical estimate. This is calculated as a function of the weight of the sample, not by a cell assay. | | | |

### Example 6

The gels of Example 3 were spread to a thickness of 5 mm and left to air dry at room temperature for 15-20 hours. Each dry gel was then stored in the same manner, in a plastic container at room temperature.

The survival rate of the micro-organisms were tested and the results are set out in the attached Table 6.

**TABLE 6**

| **Example 6** | | | |
|---|---|---|---|
| **Organism** | **Initial Concentration cfu g⁻¹** | **Survival 1 month cfu g⁻¹** | **Survival 2 months cfu g⁻¹** |
| *Serratia entomophila* | 5.44x10¹⁰ | 5.98x10⁷ | - |
| *Serratia marcescens* | 1.31x10¹⁰ | 2.42x10⁸ | 9.47x10⁶ |
| *Pseudomonas aeruginosa* | 1.09x10¹¹ | 4.71x10⁹ | 2.58x10⁷ |
| *Xanthomonas campestri* | 2.71x10¹⁰ | 1.75x10¹⁰ | 6.02x10⁹ |

## Claims

1. A method for producing a thermo-stable bio-degradable medium for storage of biological materials, said method comprising the steps of:
(a) preparing at least one blo-polymer at a concentration of between 10% and 100% (by weight) of a mixture at room temperature, said mixture being in a state selected from a solid or a suspension;
(b) preparing a concentrate of the biological materials of between 10% and 100% (by weight), said concentrate being a suspension;
(c) combining the mixture of step (a) and the preparation (b), to form a second mix; and
(d) agitating the second mix at room temperature to form a homogeneous suspension; wherein a gel matrix is formed;
and wherein the bio-polymer is selected from the group consisting of: xanthan gum; acacia gum; guar gum; gellan; or a combination thereof;
and wherein the biological material is a micro-organism.

2. A method of producing a thermo-stable biodegradable medium as claimed in claim 1 wherein said biological material is between 10% to 20% by weight in the concentrate of step (b).

3. A method of producing a thermo-stable biodegradable medium as claimed in claim 1 or claim 2 wherein, within step (c) the ratio of the mixtures of steps (a) and (b), which are combined in step (c), is in the range 1:10 to 10:1 by weight.

4. A method as claimed in any one of the preceding claims wherein the second mix is allowed to stand at room temperature for approximately 60 minutes after step (d).

5. A method of producing a thermo-stable bio-degradable medium as claimed in any one of the preceding claims wherein said method includes a further step, before step (d), but after step (c) of:
(ci) adding a bio-degradable non-toxic oil to the mix, the concentration of oil being in the range 0.1 to 10% by weight of the mix.

6. A method as claimed in claim 5 wherein the oil used in step (ci) is selected from the group consisting of: a monounsaturated oil; a refined oil; a non-refined oil; and a combination thereof.

7. A method as claimed in claim 6 wherein the oil used in step (ci) is selected from the group consisting of: olive oil; canola oil; sunflower seed oil; hydrolyzed oils; and a combination thereof.

8. A method as claimed in any one of the above claims wherein the microorganism cell concentration is in the range 10⁵ cells to 10¹² cells g⁻¹.

9. A method as claimed in any one of claims 1 to 7 wherein the biological material in the concentrate of step (b) is the form of a broth.

10. A method of producing a thermo-stable bio-degradable medium as claimed in any one of the preceding claims wherein the micro-organism is selected from the group consisting of: *Serratia, Pseudomonas, Xanthomonas, Rhizobium,* and a combination thereof.

11. A method as claimed in any one of the preceding claims wherein the biopolymer is xanthan gum which is added as a dry solid.

12. A method as claimed in any one of claims 1 to 13 wherein the biopolymer is a mixture of xanthan and acacia gum; which are added as dry solids in a ratio in the range of 1:2 to 1:6 by weight.

13. A method as claimed in any one of the preceding claims wherein more than one bio-polymer and more than one biological agent is present in the steps (a) to (c).

14. A method of producing a thermo-stable biodegradable medium as claimed in any one of the preceding claims wherein said method further includes the steps of (a) to (c) with at least one first bio-polymer; the steps of (a) to (c) with at least one second bio-polymer; and a mixing of these two mixtures by steps (c) and (d).

15. A method as claimed in any one of the preceding claims wherein more than 50% of said biological materials are viable after a storage period of 2 months.

16. A method as claimed in claim 15 wherein the temperature range of storage is 4°C to 40°C.

17. A method of producing a thermo-stable bio-degradable medium as claimed in any one of the preceding claims wherein said method includes a further step. (e) of:
(e) spreading the gel to 5-10 mm in thickness and air-drying it to a moisture content in the range 0.05% to 20% by weight.

18. A method as claimed in claim 17 wherein the moisture content is approximately 20% by weight at the end of the drying step.

## Patentansprüche

1. Verfahren zum Herstellen eines wärmestabilen, biologisch abbaubaren Mediums zum Speichern von biologischen Materialien, wobei das Verfahren folgende Schritte umfasst:
(a) Herstellen von wenigstens einem Biopolymer mit einer Konzentration von zwischen 10 % und 100 % (Gew.) eines Gemischs bei Raumtemperatur, wobei sich das Gemisch in einem Zustand befindet, der ausgewählt ist aus einem Feststoff und einer Suspension;
(b) Herstellen eines Konzentrats der biologischen Materialien von zwischen 10 % und 100 % (Gew.), wobei das Konzentrat eine Suspension ist;
(c) Kombinieren des Gemischs aus Schritt (a) und der Zubereitung (b), um ein zweites Gemisch zu bilden; und
(d) Rühren des zweiten Gemischs bei Raumtemperatur, um eine homogene Suspension zu bilden; wobei eine Gelmatrix gebildet wird;
und wobei das Biopolymer ausgewählt ist aus der Gruppe, bestehend aus Xanthangummi; Gummi arabicum; Guargummi; Gellan; und einer Kombination davon;
und wobei das biologische Material ein Mikroorganismus ist.

2. Verfahren zum Herstellen eines wärmestabilen, biologisch abbaubaren Mediums gemäß Anspruch 1, wobei das biologische Material in dem Konzentrat aus Schritt (b) zu zwischen 10 Gew.-% und 20 Gew.-% vorliegt.

3. Verfahren zum Herstellen eines wärmestabilen, biologisch abbaubaren Mediums gemäß Anspruch 1 oder Anspruch 2, wobei bei Schritt (c) das Verhältnis der Gemische aus den Schritten (a) und (b), die bei Schritt (c) kombiniert werden, im Bereich von 1:10 bis 10:1, bezogen auf das Gewicht, liegt.

4. Verfahren gemäß einem der vorstehenden Ansprüche, wobei das zweite Gemisch nach Schritt (d) etwa 60 Minuten bei Raumtemperatur stehen gelassen wird.

5. Verfahren zum Herstellen eines wärmestabilen, biologisch abbaubaren Mediums gemäß einem der vorstehenden Ansprüche, wobei das Verfahren einen zusätzlichen Schritt vor Schritt (d) aber nach Schritt (c) umfasst:
(ci) Zugeben eines biologisch abbaubaren, nichttoxischen Öls zu dem Gemisch, wobei die Konzentration des Öls im Bereich von 0,1 bis 10 Gew.-% des Gemischs beträgt.

6. Verfahren gemäß Anspruch 5, wobei das bei Schritt (ci) verwendete Öl ausgewählt ist aus der Gruppe, bestehend aus einem einfach ungesättigten Öl; einem raffinierten Öl; einem nicht-raffinierten Öl; und einer Kombination davon.

7. Verfahren gemäß Anspruch 6, wobei das bei Schritt (ci) verwendete Öl ausgewählt ist aus der Gruppe, bestehend aus Olivenöl; Rapsöl; Sonnenblumensamenöl; hydrolysierten Ölen; und einer Kombination davon.

8. Verfahren gemäß einem der vorstehenden Ansprüche, wobei die Konzentration der Zellen des Mikroorganismus im Bereich von 10⁵ Zellen bis 10¹² Zellen g⁻¹ liegt.

9. Verfahren gemäß einem der Ansprüche 1 bis 7, wobei das biologische Material in dem Konzentrat bei Schritt (b) in der Form von Brühe vorliegt.

10. Verfahren zum Herstellen eines wärmestabilen, biologisch abbaubaren Mediums gemäß einem der vorstehenden Ansprüche, wobei der Mikroorganismus ausgewählt ist aus der Gruppe, bestehend aus *Serratia, Pseudomonas, Xanthomonas, Rhizobium* und einer Kombination davon.

11. Verfahren gemäß einem der vorstehenden Ansprüche, wobei das Biopolymer Xanthangummi ist, der als trockener Feststoff zugegeben wird.

12. Verfahren gemäß einem der Ansprüche 1 bis 10, wobei Biopolymer ein Gemisch von Xanthangummi und Gummi arabicum ist, die als trockene Feststoffe in einem Verhältnis in einem Bereich von 1:2 bis 1:6, bezogen auf das Gewicht, zugegeben werden.

13. Verfahren gemäß einem der vorstehenden Ansprüche, wobei bei den Schritten (a) bis (c) mehr als ein Biopolymer und mehr als ein biologisches Mittel vorhanden ist.

14. Verfahren zum Herstellen eines wärmestabilen, biologisch abbaubaren Mediums gemäß einem der vorstehenden Ansprüche, wobei das Verfahren ferner die Schritte (a) bis (c) mit wenigstens einem ersten Biopolymer; die Schritte (a) bis (c) mit wenigstens einem zweiten Biopolymer; und Mischen dieser beiden Gemische in den Schritten (c) und (d) umfasst.

15. Verfahren gemäß einem der vorstehenden Ansprüche, wobei mehr als 50 % der biologischen Materialien nach einem Lagerungszeitraum von 2 Monaten lebensfähig sind.

16. Verfahren gemäß Anspruch 15, wobei der Temperaturbereich der Lagerung 4 °C bis 40 °C ist.

17. Verfahren zum Herstellen eines wärmestabilen, biologisch abbaubaren Mediums gemäß einem der vorstehenden Ansprüche, wobei das Verfahren einen zusätzlichen Schritt (e) umfasst:
(e) Ausbreiten des Gels auf eine Dicke von 5-10 mm und Lufttrocknen davon auf einen Feuchtegehalt in Bereich von 0,05 Gew.-% bis 20 Gew.-%.

18. Verfahren gemäß Anspruch 17, wobei der Feuchtegehalt am Ende des Trocknungsschritts etwa 20 Gew.-% beträgt.

## Revendications

1. Méthode de production d'un support thermostable biodégradable pour la conservation de matériels biologiques, ladite méthode comprenant les étapes suivantes :
(a) préparation d'au moins un biopolymère à une concentration comprise entre 10 % et 100 % (en masse) d'un mélange à température ambiante, ledit mélange étant dans un état choisi parmi un état solide et une suspension ;
(b) préparation d'un concentré des matériels biologiques compris entre 10 % et 100 % (en masse), ledit concentré étant une suspension ;
(c) combinaison du mélange de l'étape (a) et de la préparation (b), pour former un second mélange ; et
(d) agitation du second mélange à température ambiante pour former une suspension homogène ; où une matrice de gel se forme ;
et où le biopolymère est choisi dans le groupe constitué par : la gomme xanthane ; la gomme d'acacia ; la gomme de guar ; la gomme gellane ; ou l'une de leurs combinaisons ;
et où le matériel biologique est un micro-organisme.

2. Méthode de production d'un support thermostable biodégradable conforme à la revendication 1, où ledit matériel biologique est inclus à une teneur comprise entre 10 % et 20 % en masse dans le concentré de l'étape (b).

3. Méthode de production d'un milieu thermostable biodégradable conforme à la revendication 1 ou à la revendication 2 où, dans l'étape (c), le rapport des mélanges des étapes (a) et (b) qui sont combinés dans l'étape (c) est inclus dans l'intervalle compris entre 1:10 et 10:1 en masse.

4. Méthode conforme à l'une quelconque des revendications précédentes, où le second mélange est laissé à reposer à température ambiante pendant environ 60 minutes après l'étape (d).

5. Méthode de production d'un support thermostable biodégradable conforme à l'une quelconque des revendications précédentes, ladite méthode incluant une étape supplémentaire avant l'étape (d) mais après l'étape (c) :
(ci) ajout d'une huile biodégradable non toxique au mélange, la concentration d'huile étant incluse dans l'intervalle compris entre 0,1 et 10 % en masse du mélange.

6. Méthode conforme à la revendication 5 où l'huile employée dans l'étape (ci) est choisie dans le groupe constitué par une huile mono-insaturée ; une huile raffinée ; une huile non raffinée ; et l'une de leurs combinaisons.

7. Méthode conforme à la revendication 6, où l'huile employée dans l'étape (ci) est choisie dans le groupe constitué par : l'huile d'olive ; l'huile de canola ; l'huile de tournesol ; les huiles hydrolysées ; et l'une de leurs combinaisons.

8. Méthode conforme à l'une quelconque des revendications précédentes, où la concentration en cellules de micro-organismes est incluse dans l'intervalle compris entre 10⁵ cellules et 10¹² cellules g⁻¹_{.}

9. Méthode conforme à l'une quelconque des revendications 1 à 7, où le matériel biologique dans le concentré de l'étape (b) est sous la forme d'un bouillon de culture.

10. Méthode de production d'un support thermostable biodégradable conforme à l'une quelconque des revendications précédentes, où le micro-organisme est choisi dans le groupe constitué par : *Serratia, Pseudomonas, Xanthomonas, Rhizobium* et l'une de leurs combinaisons.

11. Méthode conforme à l'une quelconque des revendications précédentes, où le biopolymère est la gomme xanthane qui est ajoutée sous forme d'un solide sec.

12. Méthode conforme à l'une quelconque des revendications 1 à 10, où le biopolymère est un mélange de gomme xanthane et de gomme d'acacia, ajoutées sous forme de solides secs dans un rapport inclus dans l'intervalle compris entre 1:2 et 1:6 en masse.

13. Méthode conforme à l'une quelconque des revendications précédentes, où plus d'un biopolymère et plus d'un agent biologique sont présents dans les étapes (a) à (c).

14. Méthode de production d'un support thermostable biodégradable conforme à l'une quelconque des revendications précédentes, ladite méthode incluant en outre les étapes (a) à (c) avec au moins un premier biopolymère ; les étapes (a) à (c) avec au moins un second biopolymère ; et un mélangeage de ces deux mélanges au niveau des étapes (c) et (d).

15. Méthode conforme à l'une quelconque des revendications précédentes, où plus de 50 % desdits matériels biologiques sont viables après une période de conservation de 2 mois.

16. Méthode conforme à la revendication 15, où l'intervalle de températures de conservation est compris entre 4 °C et 40 °C.

17. Méthode de production d'un support thermostable biodégradable conforme à l'une quelconque des revendications précédentes, ladite méthode incluant une étape supplémentaire (e) :
(e) étalement du gel jusqu'à une épaisseur de 5 à 10 mm et son séchage à l'air jusqu'à une teneur en humidité incluse dans l'intervalle compris entre 0,05 % et 20 % en masse.

18. Méthode conforme à la revendication 17, où la teneur en humidité est d'environ 20 % en masse à la fin de l'étape de séchage.
